# EUROPÄISCHE PATENTSCHRIFT

(11) **EP 2 547 444 B1**
(45) Veröffentlichungstag und Bekanntmachung des Hinweises auf die Patenterteilung: **07.05.2014**
(21) Anmeldenummer: 11709705.5
(22) Anmeldetag: 18.03.2011
(51) Int. Cl.: B01J 21/06, B01J 23/22, C07C 51/265

(54) **KATALYSATOR FÜR GASPHASENOXIDATIONEN AUF DER BASIS VON SCHWEFEL- UND CALCIUMARMEM TITANDIOXID**
CATALYST FOR GAS-PHASE OXIDATIONS ON THE BASIS OF SULFUR- AND CALCIUM-POOR TITANIUM DIOXIDE
CATALYSEUR POUR OXYDATIONS EN PHASE GAZEUSE SUR LA BASE DE DIOXYDE DE TITANE PAUVRE EN SOUFRE ET EN CALCIUM

(30) Priorität: 19.03.2010 EP 10157119
(43) Veröffentlichungstag der Anmeldung: 23.01.2013
(73) Patentinhaber: BASF SE, 67056 Ludwigshafen (DE)
(72) Erfinder: ALTWASSER, Stefan, 67157 Wachenheim (DE); SCHMITT, Christine, 68163 Mannheim (DE); KARPOV, Andrey, 68159 Mannheim (DE); DOBNER, Cornelia Katharina, 67071 Ludwigshafen (DE); EWALD, Bastian, 67061 Ludwigshafen (DE); ROSOWSKI, Frank, 68239 Mannheim (DE); WILMER, Hagen, 67071 Ludwigshafen (DE)
(86) Internationale Anmeldenummer: PCT/EP2011/054092
(87) Internationale Veröffentlichungsnummer: WO 2011/113918

(56) Entgegenhaltungen:
- WO-A1-2006/053732
- WO-A1-2007/134849
- GRZYBOWSKA-SWIERKOSZ B: "Vanadia-titania catalysts for oxidation of o-xylene and other hydrocarbons", APPLIED CATALYSIS A: GENERAL, ELSEVIER SCIENCE, AMSTERDAM, NL, Bd. 157, Nr. 1-2, 11. September 1997 (1997-09-11), Seiten 263-310, XP004338200, ISSN: 0926-860X, DOI: DOI:10.1016/S0926-860X(97)00015-X in der Anmeldung erwähnt

## Beschreibung

Die Erfindung betrifft einen Katalysator für Gasphasenoxidationen, der einen inerten Träger und eine darauf aufgebrachte katalytisch aktive Masse umfasst, die Vanadiumoxid und Titandioxid enthält, ein Verfahren zu seiner Herstellung, sowie die Verwendung des Katalysators zur Herstellung von Phthalsäureanhydrid.

Eine Vielzahl von Carbonsäuren und/oder Carbonsäureanhydriden wird technisch durch die katalytische Gasphasenoxidation von aromatischen Kohlenwasserstoffen, wie Benzol, den Xylolen, Naphthalin, Toluol oder Durol, in Festbettreaktoren hergestellt. Man kann auf diese Weise z. B. Benzoesäure, Maleinsäureanhydrid, Phthalsäureanhydrid (PSA), Isophthalsäure, Terephthalsäure oder Pyromellithsäureanhydrid erhalten. Im Allgemeinen leitet man ein Gemisch aus einem sauerstoffhaltigen Gas und dem zu oxidierenden Ausgangsmaterial durch Rohre, in denen sich eine Schüttung eines Katalysators befindet. Zur Temperaturregelung sind die Rohre von einem Wärmeträgermedium, beispielsweise einer Salzschmelze, umgeben.

Als Katalysatoren haben sich für diese Oxidationsreaktionen so genannte Schalenkatalysatoren bewährt, bei denen die katalytisch aktive Masse schalenförmig auf einem inerten Trägermaterial, wie Steatit, aufgebracht ist. Als katalytisch aktiver Bestandteil der katalytisch aktiven Masse dieser Schalenkatalysatoren dient im Allgemeinen neben Titandioxid Vanadiumpentoxid. Des weiteren können in der katalytisch aktiven Masse in geringen Mengen eine Vielzahl anderer oxidischer Verbindungen enthalten sein, die als Promotoren die Aktivität und Selektivität des Katalysators beeinflussen.

Der Einfluss von Verunreinigungen im eingesetzten Titandioxid ist im Stand der Technik untersucht worden. Grzybowska-Swierkosz erwähnt in Appl. Catal. A: Gen. 157 (1997) 263-310, dass Verunreinigungen auf der Oberfläche handelsüblicher Titandioxidpigmente die Struktur oberflächengebundener OH-Gruppen beeinflussen können.

Die WO 2007/134849 beschreibt die Verwendung bestimmter Titandioxide zur Herstellung eines Katalysators zur Oxidation von o-Xylol zu Phthalsäureanhydrid. Das Titandioxid soll weniger als 1000 ppm Schwefel, weniger als 300 ppm Phosphor und mehr als 500 ppm Niob enthalten.

Garcin et al. (Catalysis Today 20 (1994) 7-10) nutzt ein Titandioxid für einen PSA-Katalysator, das durch folgende Verunreinigungen charakterisiert ist: 0,12 Gew.-% Sulfat, < 0,005 Gew.-% SiO₂, < 0,005 Gew.-% Al₂O₃, 0,04 Gew.-% K₂O, < 0,002 Gew.-% Sb₂O₃, < 0,22 Gew.-% Nb₂O₅, 0,02 Gew.-% ZrO₂, < 0,002 Gew.-% SnO₂, 27 ppm Fe, 0,24 Gew.-% P₂O₅ und 0,023 Gew.-% CaO (entsprechend 164 ppm Ca).

In der EP-A 539878 ist angegeben, dass die Nebenkomponenten Fe, Zn, Al, Mn, Cr, Ca, und Pb im Titandioxid nicht stören, so lange deren Gesamtmenge nicht mehr als 0,5 Gew.-% (als Metalloxid), bezogen auf die Menge an Titandioxid, beträgt.

Es besteht ein ständiger Bedarf an Katalysatoren für Gasphasenoxidationen, die einen möglichst hohen Umsatz bei hoher Selektivität aufweisen.

Der Erfindung liegt die Aufgabe zugrunde, einen Katalysator für Gasphasenoxidationen anzugeben, der eine höhere Aktivität und/oder Selektivität aufweist und so höhere Ausbeuten des gewünschten Zielprodukts, z. B. Phthalsäureanhydrid, ermöglicht.

Die Aufgabe wird gelöst durch einen Katalysator für Gasphasenoxidationen, der einen inerten Träger und eine darauf aufgebrachte katalytisch aktive Masse umfasst, die Vanadiumoxid und Titandioxid enthält, wobei das Titandioxid einen Gehalt an Schwefelverbindungen, berechnet als S, von weniger als 1000 ppm und einen Gehalt an Calciumverbindungen, berechnet als Ca, von weniger als 100 ppm und eine BET-Oberfläche von 15 bis 60 m²/g aufweist.

Die Erfindung betrifft außerdem ein Verfahren zur Herstellung eines Katalysators für Gasphasenoxidationen, bei dem man auf einen inerten Träger eine Suspension von Titandioxid- und Vanadiumoxid-Partikeln aufbringt, wobei das Titandioxid einen Gehalt an Schwefelverbindungen, berechnet als S, von weniger als 1000 ppm und einen Gehalt an Calciumverbindungen, berechnet als Ca, von weniger als 100 ppm und eine BET-Oberfläche von 15 bis 60 m²/g aufweist. In einer bevorzugten Ausführungsform behandelt man vor dem Aufbringen zumindest einen Teil des Titandioxids mit einem wässrigen Medium unter hydrothermalen Bedingungen.

Das erfindungsgemäß verwendete Titandioxid weist einen bestimmten Gehalt an Schwefelverbindungen und Calciumverbindungen auf. Die Bestimmung der chemischen Verunreinigungen des TiO₂, insbesondere der Gehalte an S, Ca, P und Nb, erfolgt nach DIN ISO 9964-3. Dabei werden die Gehalte mittels ICP-AES (Atomic Emission Spectroscopy with Inductively Coupled Plasma) bestimmt.

Werden Gemische verschiedener Titandioxide verwendet, bestimmt sich der Gehalt an Schwefelverbindungen und Calciumverbindungen als gewichteter Mittelwert der Gehalte der einzelnen Titandioxide im Gemisch. Die Verwendung von Gemischen verschiedener Titandioxide kann z. B. geeignet sein, um einen gewünschten Wert der BET-Oberfläche einzustellen, indem man ein Titandioxid hoher BET-Oberfläche und ein Titandioxid niedriger BET-Oberfläche in bestimmten Anteilen mischt.

In bevorzugten Ausführungsformen weist das Titandioxid einen Gehalt an Schwefelverbindungen von weniger als 500 ppm, insbesondere weniger als 400 ppm, z. B. 100 bis 300 ppm, auf.

In bevorzugten Ausführungsformen weist das Titandioxid einen Gehalt an Calciumverbindungen, berechnet als Ca, von weniger als 80 ppm, z. B. 50 bis 75 ppm, auf.

In bevorzugten Ausführungsformen weist das Titandioxid darüber hinaus einen Gehalt an Phosphorverbindungen, berechnet als P, von weniger als 1000 ppm, insbesondere weniger als 500 ppm, z. B. 100 bis 300 ppm, auf.

In bevorzugten Ausführungsformen weist das Titandioxid darüber hinaus einen Gehalt an Niobverbindungen, berechnet als Nb, von mehr als 200 ppm, insbesondere mehr als 500 ppm, z. B. 600 bis 2000 ppm, auf.

Geeignete TiO₂-Materialien sind entweder handelsüblich oder können vom Fachmann nach Standard-Verfahren erhalten werden, soweit bei der Synthese darauf geachtet wird, dass die verwendeten Rohstoffe entsprechend geringe Verunreinigungen an Schwefel und Calcium enthalten. Alternativ kann man auch von TiO₂-Materialien mit höherem S bzw. Ca-Gehalt ausgehen und durch geeignete Behandlung, z.B. Auslaugung, erfindungsgemäß geeignete Gehalte eingestellt werden.

In einer geeigneten Ausführungsform wird zumindest ein Teil des Titandioxids mit einem wässrigen Medium unter hydrothermalen Bedingungen behandelt. Im Rahmen der vorliegenden Erfindung sind unter hydrothermalen Bedingungen Temperaturen von wenigstens 80°C sowie Drücke oberhalb Atmosphärendruck (größer 1 atm) zu verstehen. Bevorzugt sind Temperaturen zwischen 120 und 500 °C, besonders bevorzugt zwischen 180 und 300 °C und Drücke oberhalb von Atmosphärendruck, z. B. der Eigendruck, der sich bei der angegebenen Temperatur in einem geschlossenen Gefäß einstellt. Die Behandlung unter hydrothermalen Bedingungen kann sich z. B. über 15 bis 24 Stunden, vorzugsweise 30 min bis 6 Stunden, erstrecken. Als wässriges Medium eignet sich vor allem Wasser, z. B. entmineralisiertes oder bidestilliertes Wasser, oder verdünnte Säuren oder Basen wie 0,1 - 1 molare Salpetersäure, oder 1 molares Ammoniakwasser. Anschließend wird das Titandioxidmaterial von dem wässrigen Medium abgetrennt, z. B. durch Filtration, gegebenenfalls gewaschen und getrocknet. Die Behandlung kann gewünschtenfalls wiederholt werden.

Üblicherweise wird das Titandioxid in der Anatas-Form verwendet. Das Titandioxid weist vorzugsweise eine BET-Oberfläche von 15 bis 45 m²/g, besonders bevorzugt 13 bis 28 m²/g auf. Das eingesetzte Titandioxid kann aus einem einzelnen Titandioxid oder einem Gemisch von Titandioxiden bestehen. In letzterem Fall bestimmt sich der Wert der BET-Oberfläche als gewichteter Mittelwert der Beiträge der einzelnen Titandioxide. Das eingesetzte Titandioxid besteht z. B. vorteilhaft aus einem Gemisch eines TiO₂ mit einer BET-Oberfläche von 5 bis 15 m²/g und eines TiO₂ mit einer BET-Oberfläche von 15 bis 50 m²/g.

Vorzugsweise enthält die katalytisch aktive Masse, bezogen auf die Gesamtmenge der katalytisch aktiven Masse, 1 bis 40 Gew.-% Vanadiumoxid, berechnet als V₂O₅, und 60 bis 99 Gew.-% Titandoxid, berechnet als TiO₂. Die katalytisch aktive Masse kann in bevorzugten Ausführungsformen daneben bis zu 1 Gew.-% einer Cäsiumverbindung, berechnet als Cs, bis zu 1 Gew.-% einer Phosphorverbindung, berechnet als P, und bis zu 10 Gew.-% Antimonoxid, berechnet als Sb₂O₃ enthalten. Alle Angaben zur Zusammensetzung der katalytisch aktiven Masse beziehen sich auf deren calzinierten Zustand, z. B. nach Calcination des Katalysators für eine Stunde bei 450 °C.

Als Vanadiumquelle eignen sich besonders Vanadiumpentoxid oder Ammoniummetavanadat.

Als Antimonquelle eignen sich verschiedene Antimonoxide, insbesondere Antimontrioxid. Im Allgemeinen verwendet man Antimontrioxid mit einer mittleren Teilchengröße (Maximum der Teilchengrößenverteilung) von 0,1 bis 10 µm. Besonders bevorzugt verwendet man als Quelle des Antimonoxids im ersten Katalysator teilchenförmiges Antimontrioxid mit einer mittleren Teilchengröße von 0,5 bis 5 µm, insbesondere 1 bis 4 µm. Als Phosphorquelle kommen insbesondere Phosphorsäure, phosphorige Säure, hypophosphorige Säure, Ammoniumphosphat oder Phosphorsäureester und vor allem Ammoniumdihydrogenphosphat in Betracht. Als Quellen von Cäsium kommen das Oxide oder Hydroxid oder die thermisch in das Oxid überführbare Salze wie Carboxylate, insbesondere das Acetat, Malonat oder Oxalat, Carbonat, Hydrogencarbonat, Sulfat oder Nitrat in Betracht.

Neben den fakultativen an Zusätzen Cäsium und Phosphor können in der katalytisch aktiven Masse in geringen Mengen eine Vielzahl anderer oxidischer Verbindungen enthalten sein, die als Promotoren die Aktivität und Selektivität des Katalysators beeinflussen, beispielsweise indem sie seine Aktivität absenken oder erhöhen. Als solche Promotoren seien beispielhaft die Alkalimetalloxide, insbesondere außer dem genannten Cäsiumoxid, Lithium-, Kalium- und Rubidiumoxid, Thallium(I)oxid, Aluminiumoxid, Zirkoniumoxid, Eisenoxid, Nickeloxid, Kobaltoxid, Manganoxid, Zinnoxid, Silberoxid, Kupferoxid, Chromoxid, Molybdänoxid, Wolframoxid, Iridiumoxid, Tantaloxid, Nioboxid, Arsenoxid, Antimonoxid, Ceroxid genannt.

Ferner kommen von den genannten Promotoren noch bevorzugt als Zusätze die Oxide von Niob und Wolfram in Mengen von 0,01 bis 0,50 Gew.-%, bezogen auf die katalytisch wirksame Masse in Betracht.

Als inertes Trägermaterial können praktisch alle Trägermaterialien des Standes der Technik, wie sie vorteilhaft bei der Herstellung von Schalenkatalysatoren für die Oxidation aromatischer Kohlenwasserstoffe zu Aldehyden, Carbonsäuren und/oder Carbonsäureanhydriden eingesetzt werden, Verwendung finden, beispielsweise Quarz (SiO₂), Porzellan, Magnesiumoxid, Zinndioxid, Siliciumcarbid, Rutil, Tonerde (Al₂O₃), Aluminiumsilikat, Steatit (Magnesiumsilikat), Zirkoniumsilikat, Cersilikat oder Mischungen dieser Trägermaterialien. Das Trägermaterial ist in der Regel nicht-porös. Der Ausdruck "nicht-porös" ist dabei im Sinne von "bis auf technisch unwirksame Mengen an Poren nicht-porös" zu verstehen, da technisch unvermeidlich eine geringe Anzahl Poren im Trägermaterial, das idealerweise keine Poren enthalten sollte, vorhanden sein können. Als vorteilhafte Trägermaterialien sind insbesondere Steatit und Siliciumcarbid hervorzuheben. Die Form des Trägermaterials ist für die erfindungsgemäßen Präkatalysatoren und Schalenkatalysatoren im Allgemeinen nicht kritisch. Beispielsweise können Katalysatorträger in Form von Kugeln, Ringen, Tabletten, Spiralen, Röhren, Extrudaten oder Splitt verwendet werden. Die Dimensionen dieser Katalysatorträger entsprechen denen üblicherweise zur Herstellung von Schalenkatalysatoren für die Gasphasenpartialoxidation von aromatischen Kohlenwasserstoffen verwendeten Katalysatorträgern. Bevorzugt wird Steatit in Form von Kugeln mit einem Durchmesser von 3 bis 6 mm oder von Ringen mit einem äußeren Durchmesser von 5 bis 9 mm und einer Länge von 3 bis 8 mm und einer Wandstärke von 1 bis 2 mm verwendet.

Das Aufbringen der Schicht(en) des Schalenkatalysators erfolgt zweckmäßigerweise durch Aufsprühen einer Suspension von TiO₂ und V₂O₅, die gegebenenfalls Quellen der oben genannten Promotorelemente enthält, auf den fluidisierten Träger. Vor der Beschichtung wird die Suspension vorzugsweise ausreichend lange, z. B. 2 bis 30 Stunden, insbesondere 12 bis 25 Stunden, gerührt, um Agglomerate der suspendierten Feststoffe aufzubrechen und eine homogene Suspension zu erhalten. Die Suspension hat typischerweise einen Feststoffgehalt von 20 bis 50 Gew.-%. Das Suspensionsmedium ist im Allgemeinen wässrig, z. B. Wasser selbst oder ein wässriges Gemisch mit einem wassermischbaren organischen Lösungsmittel, wie Methanol, Ethanol, Isopropanol, Formamid und dergleichen.

In der Regel werden der Suspension organische Binder, bevorzugt Copolymere, vorteilhaft in Form einer wässrigen Dispersion, von Acrylsäure/Maleinsäure, Vinylacetat/Vinyllaurat, Vinylacetat/Acrylat, Styrol/Acrylat sowie Vinylacetat/Ethylen zugesetzt. Die Binder sind als wässrige Dispersionen handelsüblich, mit einem Feststoffgehalt von z. B. 35 bis 65 Gew.-%. Die eingesetzte Menge solcher Binderdispersionen beträgt im Allgemeinen 2 bis 45 Gew.-%, vorzugsweise 5 bis 35 Gew.-%, besonders bevorzugt 7 bis 20 Gew.-%, bezogen auf das Gewicht der Suspension.

Der Träger wird in z. B. einer Wirbelschicht- bzw. Fließbettapparatur in einem aufsteigenden Gasstrom, insbesondere Luft, fluidisiert. Die Apparate bestehen meist aus einem konischen oder kugelförmigen Behälter, bei dem das fluidisierende Gas von unten oder von oben über ein Tauchrohr eingeführt wird. Die Suspension wird über Düsen von oben, seitlich oder von unten in die Wirbelschicht eingesprüht. Vorteilhaft ist der Einsatz eines mittig bzw. konzentrisch um das Tauchrohr angeordneten Steigrohrs. Innerhalb des Steigrohres herrscht eine höhere Gasgeschwindigkeit, die die Trägerpartikel nach oben transportiert. Im äußeren Ring liegt die Gasgeschwindigkeit nur wenig oberhalb der Lockerungsgeschwindigkeit. So werden die Partikel kreisförmig vertikal bewegt. Eine geeignete Fließbettvorrichtung ist z. B. in der DE-A 4006935 beschrieben.

Bei der Beschichtung des Katalysatorträgers mit der katalytisch aktiven Masse werden im Allgemeinen Beschichtungstemperaturen von 20 bis 500 °C angewandt, wobei die Beschichtung unter Atmosphärendruck oder unter reduziertem Druck erfolgen kann. Im Allgemeinen erfolgt die Beschichtung bei 0 °C bis 200 °C, vorzugsweise bei 20 bis 150 °C, insbesondere bei 60 bis 120 °C durchgeführt.

Die katalytisch aktive Masse kann auch in zwei oder mehreren Schichten aufgebracht sein, wobei z. B. die innere Schicht oder die innere Schichten einen Antimonoxidgehalt von bis zu 15 Gew.-% und die äußere Schicht einen um 50 bis 100% verringerten Antimonoxidgehalt aufweisen. Dabei ist in der Regel die innere Schicht des Katalysators phosphorhaltig und die äußere Schicht phosphorarm oder phosphorfrei.

Die Schichtdicke der katalytisch aktiven Masse beträgt in der Regel 0,02 bis 0,2 mm, vorzugsweise 0,05 bis 0,15 mm. Der Aktivmasseanteil am Katalysator beträgt üblicherweise 5 bis 25 Gew.-%, meist 7 bis 15 Gew.-%.

Durch thermische Behandlung des so erhaltenen Präkatalysators bei Temperaturen über 200 bis 500 °C entweicht das Bindemittel durch thermische Zersetzung und/oder Verbrennung aus der aufgetragenen Schicht. Vorzugsweise erfolgt die thermische Behandlung in situ im Gasphasenoxidationsreaktor.

Die erfindungsgemäßen Katalysatoren eignen sich generell zur Gasphasenoxidation aromatischer C₆- bis C₁₀-Kohlenwasserstoffe, wie Benzol, den Xylolen, Toluol, Naphthalin oder Durol (1,2,4,5-Tetramethylbenzol) zu Carbonsäuren und/oder Carbonsäureanhydriden wie Maleinsäureanhydrid, Phthalsäureanhydrid, Benzoesäure und/oder Pyromellithsäuredianhydrid.

Eine Ausführungsform der Erfindung betrifft ein Verfahren zur Herstellung von Phthalsäureanhydrid, bei dem man einen Gasstrom, der molekularen Sauerstoff sowie o-Xylol, Naphthalin oder Gemische davon enthält, in Kontakt mit einem erfindungsgemäßen Katalysator bringt.

Zu diesem Zweck werden die erfindungsgemäß hergestellten Katalysatoren in von außen auf die Reaktionstemperatur, beispielsweise mittels Salzschmelzen, thermostatisierte Reaktionsrohre gefüllt und über die so bereitete Katalysatorschüttung das Reaktionsgas Temperaturen von im allgemeinen 300 bis 450 °C, vorzugsweise von 320 bis 420 °C und besonders bevorzugt von 340 bis 400 °C und bei einem Überdruck von im allgemeinen 0,1 bis 2,5 bar, vorzugsweise von 0,3 bis 1,5 bar mit einer Raumgeschwindigkeit von im allgemeinen 750 bis 5000 h⁻¹ geleitet.

Das dem Katalysator zugeführte Reaktionsgas wird im allgemeinen durch Vermischen von einem molekularen Sauerstoff enthaltenden Gas, das außer Sauerstoff noch geeignete Reaktionsmoderatoren und/oder Verdünnungsmittel, wie Dampf, Kohlendioxid und/oder Stickstoff, enthalten kann, mit dem zu oxidierenden, aromatischen Kohlenwasserstoff erzeugt, wobei das molekularen Sauerstoff enthaltende Gas im allgemeinen 1 bis 100 mol-%, vorzugsweise 2 bis 50 mol-% und besonders bevorzugt 10 bis 30 mol-% Sauerstoff, 0 bis 30 mol-%, vorzugsweise 0 bis 10 mol-% Wasserdampf sowie 0 bis 50 mol-%, vorzugsweise 0 bis 1 mol-% Kohlendioxid, Rest Stickstoff, enthalten kann. Zur Erzeugung des Reaktionsgases wird das molekularen Sauerstoff enthaltende Gas im Allgemeinen mit 30 g bis 150 g je Nm³ Gas des zu oxidierenden, aromatischen Kohlenwasserstoffs beschickt.

Es hat sich als besonders vorteilhaft erwiesen, wenn in der Katalysatorschüttung Katalysatoren eingesetzt werden, die sich in ihrer katalytischen Aktivität und/oder chemischen Zusammensetzung ihrer Aktivmasse unterscheiden. Vorzugsweise wird bei Anwendung zweier Reaktionszonen in der ersten, also zum Gaseintritt des Reaktionsgases hin gelegenen Reaktionszone, ein Katalysator eingesetzt, der in Vergleich zum Katalysator, welcher sich in der zweiten, also zum Gasaustritt hin gelegenen Reaktionszone, befindet, eine etwas geringere katalytische Aktivität hat. Im Allgemeinen wird die Umsetzung durch die Temperatureinstellung so gesteuert, dass in der ersten Zone der größte Teil der im Reaktionsgas enthaltenen aromatischen Kohlenwasserstoff bei maximaler Ausbeute umgesetzt wird. Bevorzugt werden drei- bis fünflagige Katalysatorsysteme verwendet, insbesondere drei- und vierlagige Katalysatorsysteme.

In einer bevorzugten Ausführungsform eines dreilagigen Katalysatorsystems weisen die Katalysatoren folgende Zusammensetzung auf:
- für die erste, oberste Lage (Lage KL1):
   7 bis 10 Gew.-% Aktivmasse bezogen auf den gesamten Katalysator, wobei diese Aktivmasse:
   6 bis 11 Gew.-% Vanadium (berechnet als V₂O₅)
   0 bis 6 Gew.-% Antimontrioxid
   0,1 bis 1 Gew.-% eines Alkali (ber. als Alkalimetall), insbesondere Cäsiumoxid und als Rest auf 100 Gew.-% Titandioxid in Anatasmodifikation mit einer BET-Oberfläche von 10 bis 25 m²/g enthält;
- für die zweite, mittlere Lage (Lage KL2):
   7 bis 12 Gew.-% Aktivmasse bezogen auf den gesamten Katalysator, wobei diese Aktivmasse:
   5 bis 13 Gew.-% Vanadium (berechnet als V₂O₅)
   0 bis 6 Gew.-% Antimontrioxid
   0 bis 0,4 Gew.-% eines Alkali (ber. als Alkalimetall), insbesondere Cäsiumoxid
   0 bis 0,4 Gew.-% Phosphorpentoxid (berechnet als P)
   und als Rest auf 100 Gew.-% Titandioxid in Anatasmodifikation mit einer BET-Oberfläche von 15 bis 25 m²/g enthält;
- für die dritte, unterste Lage (Lage KL3):
   8 bis 12 Gew.-% Aktivmasse bezogen auf den gesamten Katalysator, wobei diese Aktivmasse:
   5 bis 30 Gew.-% Vanadium (berechnet als V₂O₅)
   0 bis 6 Gew.-% Antimontrioxid
   0 bis 0,3 Gew.-% eines Alkali (ber. als Alkalimetall), insbesondere Cäsiumoxid
   0,05 bis 0,4 Gew.-% Phosphorpentoxid (berechnet als P)
   und als Rest auf 100 Gew.-% Titandioxid, in Anatasmodifikation mit einer BET-Oberfläche von 15 bis 30 m²/g enthält.

In einer bevorzugten Ausführungsform eines vierlagigen Katalysatorsystems weisen die Katalysatoren folgende Zusammensetzung auf:
- für die erste Lage (Lage KL1):
   7 bis 10 Gew.-% Aktivmasse bezogen auf den gesamten Katalysator, wobei diese Aktivmasse:
   6 bis 11 Gew.-% Vanadium (berechnet als V₂O₅)
   0 bis 6 Gew.-% Antimontrioxid
   0,1 bis 1 Gew.-% eines Alkali (ber. als Alkalimetall), insbesondere Cäsiumoxid und als Rest auf 100 Gew.-% Titandioxid in Anatasmodifikation mit einer BET-Oberfläche von 5 bis 20 m²/g enthält;
- für die zweite Lage (Lage KL2):
   7 bis 12 Gew.-% Aktivmasse bezogen auf den gesamten Katalysator, wobei diese Aktivmasse:
   4 bis 15 Gew.-% Vanadium (berechnet als V₂O₅)
   0 bis 6 Gew.-% Antimontrioxid
   0,1 bis 1 Gew.-% eines Alkali (ber. als Alkalimetall), insbesondere Cäsiumoxid
   0 bis 0,4 Gew.-% Phosphorpentoxid (berechnet als P)
   und als Rest auf 100 Gew.-% Titandioxid in Anatasmodifikation mit einer BET-Oberfläche von 10 bis 25 m²/g enthält;
- für die dritte Lage (Lage KL3):
   7 bis 12 Gew.-% Aktivmasse bezogen auf den gesamten Katalysator, wobei diese Aktivmasse:
   5 bis 15 Gew.-% Vanadium (berechnet als V₂O₅)
   0 bis 6 Gew.-% Antimontrioxid
   0 bis 0,4 Gew.-% eines Alkali (ber. als Alkalimetall), insbesondere Cäsiumoxid
   0 bis 0,4 Gew.-% Phosphorpentoxid (berechnet als P)
   und als Rest auf 100 Gew.-% Titandioxid in Anatasmodifikation mit einer BET-Oberfläche von 15 bis 25 m²/g enthält;
- für die vierte Lage (Lage KL4):
   8 bis 12 Gew.-% Aktivmasse bezogen auf den gesamten Katalysator, wobei diese Aktivmasse:
   5 bis 30 Gew.-% Vanadium (berechnet als V₂O₅)
   0 bis 6 Gew.-% Antimontrioxid
   0,05 bis 0,4 Gew.-% Phosphorpentoxid (berechnet als P)
   und als Rest auf 100 Gew.-% Titandioxid in Anatasmodifikation mit einer BET-Oberfläche von 15 bis 30 m²/g enthält.

Allgemein können die Katalysatorlagen KL1, KL2, KL3 und/oder KL4 auch so angeordnet sein, dass sie jeweils aus zwei oder mehreren Lagen bestehen. Diese Zwischenlagen haben vorteilhaft intermediäre Katalysatorzusammensetzungen.

Anstelle von gegeneinander abgegrenzter Lagen der verschiedenen Katalysatoren kann auch ein quasi-kontinuierlicher Übergang der Lagen und ein quasi-gleichmässiger Anstieg der Aktivität dadurch bewirkt werden, dass man beim Übergang von einer Lage zur nächsten Lage eine Zone mit einer Vermischung der aufeinander folgenden Katalysatoren vornimmt.

Die Schüttungslänge der ersten Katalysatorlage macht vorzugsweise mehr als 30 bis 80 % der gesamten Katalysatorfüllhöhe im Reaktor aus. Die Schüttungshöhe der ersten beiden, bzw. der ersten drei Katalysatorlagen macht vorteilhaft mehr als 60 bis 95 % der gesamten Katalysatorfüllhöhe aus. Typische Reaktoren weisen eine Füllhöhe von 250 cm bis 350 cm auf. Die Katalysatorlagen können auch gegebenen-falls auf mehrere Reaktoren verteilt werden.

Gewünschtenfalls kann man für die Phthalsäureanhydridherstellung noch einen nachgeschalteten Finishing-Reaktor vorsehen, wie er beispielsweise in der DE-A 198 07 018 oder DE-A 20 05 969 beschrieben ist. Als Katalysator verwendet man dabei im Vergleich zum Katalysator der letzten Schicht vorzugsweise einen noch aktiveren Katalysator.

Wird die PSA-Herstellung mit den erfindungsgemäßen Katalysatoren unter Anwendung mehrerer Reaktionszonen, in denen sich unterschiedliche Katalysatoren befinden, durchgeführt, so können in allen Reaktionszonen die neuen Schalenkatalysatoren eingesetzt werden. Es können aber im allgemeinen bereits erhebliche Vorteile gegenüber herkömmlichen Verfahren erzielt werden, wenn nur in einer der Reaktionszonen der Katalysatorschüttung, beispielsweise der ersten Reaktionszone, oder den ersten beiden Reaktionszonen, erfindungsgemäße Schalenkatalysator verwendet werden und in den übrigen Reaktionszonen auf herkömmliche Weise hergestellte Schalenkatalysatoren benutzt werden. In der (den) ersten Reaktionszone(n) herrschen im Vergleich zu den stromabwärts gelegenen Reaktionszonen höhere Hotspot-Temperaturen; hier wird der Hauptteil des Ausgangskohlenwasserstoffs zu dem gewünschten Oxidationsprodukt und/oder Intermediaten oxidiert, so dass die Vorteile der erfindungsgemäßen Katalysatoren in der ersten Stufe oder der ersten und zweiten Stufe besonders zum Tragen kommen. Vorzugsweise kommen in wenigstens 50 % der gesamten Schüttungslänge (in Strömungsrichtung des gasförmigen Stroms) erfindungsgemäße Katalysatoren zum Einsatz.

Die Erfindung wird durch die folgenden Beispiele näher veranschaulicht.

### Katalysatorherstellung

Es wurden folgende Titandioxide eingesetzt (alle Titandioxide wiesen AnatasModifikation auf):

| Bezeichnung | BET-Oberfläche [m²/g] | S [ppm] | Nb [ppm] | P [ppm] | Ca [ppm] |
|---|---|---|---|---|---|
| A | 27 | 2400 | 1000 | 100 | 240 |
| B* | 25 | 360 | n.b. | 100 | 70 |
| C | 16 | 2400 | 1000 | 250 | 250 |
| D | 16 | 2400 | 1000 | 250 | 100 |
| E | 16 | 500 | 1000 | 250 | 250 |
| F | 16 | 300 | 1000 | 250 | 80 |
| G | 31 | 2600 | 1000 | 250 | 250 |
| H | 31 | 2600 | 1000 | 250 | 100 |
| I | 31 | 500 | 1000 | 250 | 250 |
| J | 31 | 900 | 1000 | 250 | 100 |

| | | | | | |
|---|---|---|---|---|---|
| n.b. nicht bestimmt | | | | | |

*Titandioxid B wurde aus Titandioxid A durch Nachbehandlung unter hydrothermalen Bedingungen hergestellt: 358 g Titandioxid A wurden in 1099 g Wasser unter Rühren suspendiert. Diese Suspension wurde in einen Autoklav überführt und für 72 Stunden bei 300 °C mit 370 U/min gerührt. Die erhaltene Suspension wurde abfiltriert und der Nutschkuchen mit 2 Litern Wasser gewaschen sowie 16 Stunden bei 80 °C und einem Druck von unter 100 mbar getrocknet. Der Schwefel- und Calciumgehalt von Titandioxid B wurden durch die Vorbehandlung signifikant reduziert.

### Katalysator A (nicht erfindungsgemäß)

1000 g Steatitkugeln (3,5-4,5 mm Durchmesser) wurden in einem Wirbelschichtcoater mit 14,3 g organischem Binder (Copolymer aus Acrylsäure und Maleinsäure, Gewichtsverhältnis = 75:25), und einer Suspension aus 13,06 g Vanadiumpentoxid, 34,79 g Oxalsäure, 5,64 g Antimontrioxid, 1,029 g Ammoniumdihydrogenphosphat, 0,94 g Cäsiumsulfat, 175,93 g Wasser, 36,28 g Formamid und 113,38 g Titandioxid A beschichtet. Die so hergestellte Aktivmasse enthält durchschnittlich 0,21 Gew.-% Phosphor (berechnet als P), 9,8 Gew.-% Vanadiumpentoxid (berechnet als V₂O₅), 4,2 Gew.-% Antimontrioxid (berechnet als Sb₂O₃), 0,52 Gew.-% Cäsium (berechnet als Cs) und 85,25 Gew.-% Titandioxid (berechnet als TiO₂). Der so hergestellte beschichtete Katalysator wurde anschließend mit 14,4 g organischem Binder (Copolymer aus Acrylsäure und Maleinsäure, Gewichtsverhältnis = 75:25) und einer Suspension aus 6,97 g Vanadiumpentoxid, 18,83 g Oxalsäure, 0,94 g Cäsiumsulfat, 178,74 g Wasser, 49,43 g Formamid und 125,34 g Titandioxid A beschichtet. Die so hergestellte zweite Aktivmassenschicht enthält durchschnittlich 5,2 Gew.-% Vanadiumpentoxid (berechnet als V₂O₅), 0,52 Gew.-% Cäsium (berechnet als Cs) und 94,24 Gew.-% Titandioxid (berechnet als Ti02). Insgesamt wurde mit beiden Schichten ein Gesamtaktivmassengehalt von 8,51 Gew.-% erzielt.

### Katalysator B (erfindungsgemäß)

1000 g Steatitkugeln (3,5-4,5 mm Durchmesser) wurden in einem Wirbelschichtcoater mit 16,2 g organischem Binder (Copolymer aus Acrylsäure und Maleinsäure, Gewichtsverhältnis = 75:25), und einer Suspension aus 8,50 g Vanadiumpentoxid, 22,64 g Oxalsäure, 3,68 g Antimontrioxid, 0,67 g Ammoniumdihydrogenphosphat, 0,62 g Cäsiumsulfat, 121,33 g Wasser, 25,03 g Formamid und 78,22 g Titandioxid B beschichtet. Die so hergestellte Aktivmasse enthält durchschnittlich 0,20 Gew.-% Phosphor (berechnet als P), 9,34 Gew.-% Vanadiumpentoxid (berechnet als V₂O₅), 4,0 Gew.-% Antimontrioxid (berechnet als Sb₂O₃), 0,50 Gew.-% Cäsium (berechnet als Cs) und 85,96 Gew.-% Titandioxid (berechnet als TiO₂). Der so hergestellte beschichtete Katalysator wurde anschließend mit 17,8 g organischem Binder (Copolymer aus Acrylsäure und Maleinsäure, Gewichtsverhältnis = 75:25), und einer Suspension aus 4,58 g Vanadiumpentoxid, 12,37 gOxalsäure,6,2 g Cäsiumsulfat, 122,73 g Wasser, 33,91 g Formamid und 85,97 g Titandioxid B beschichtet. Die so hergestellte zweite Aktivmassenschicht enthält durchschnittlich 5,03 Gew.-% Vanadiumpentoxid (berechnet als V₂O₅), 0,50 Gew.-% Cäsium (berechnet als Cs) und 94,47 Gew.-% Titandioxid (berechnet als TiO₂). Insgesamt wurde mit beiden Schichten ein Gesamtaktivmassengehalt von 9,74 Gew.-% erzielt. Die Zusammensetzung der Aktivmassen bei Katalysator B wurde so gewählt, dass bei Katalysator A und Katalysator B jeweils die gleiche durchschnittliche (Durchschnitt beider Aktivmassenschichten) Anzahl an Monolagen Vanadiumpentoxid aufgebracht wurden. Pro m² BET-Oberfläche des eingesetzten Titandioxid wurden dazu 0,15 Gew.-% Vanadiumpentoxid aufgebracht.

Herstellung von Katalysatorlage KL1CG (KL1 mit Titandioxid C und G, BET-Oberfläche der Titandioxidmischung = 16,15 m²/g):
3,38 g Cäsiumcarbonat, 649,6 g Titandioxid C, 6,58 g Titandioxid G, 51,37 g Vanadiumpentoxid und 13,15 g Antimontrioxid wurden in 1877 g demineralisiertem Wasser suspendiert und 18 Stunden gerührt, um eine homogene Verteilung zu erzielen. Zu dieser Suspension wurden 77,7 g organischer Binder (Copolymer aus Vinylacetat und Vinyllaurat in Form einer 50 Gew.-%igen wässrigen Dispersion) zugegeben. In einer Fließbettapparatur wurden 660 g dieser Suspension auf 2 kg Steatit (Magnesiumsilikat) in Form von Ringen mit Abmessungen von 7 mm x 7 mm x 4 mm aufgesprüht und getrocknet. Nach Kalzination des Katalysators für eine Stunde bei 450 °C betrug die auf die Steatitringe aufgebrachte Aktivmasse 9,1 %. Die analysierte Zusammensetzung der Aktivmasse bestand aus 7,1 % V₂O₅, 1,8 % Sb₂O₃, 0,38 % Cs, Rest TiO₂.

Katalysator KL2CG (BET-Oberfläche der Titandioxidmischung = 18,25 m²/g):
Die Herstellung erfolgte analog zur Herstellung von KL1 unter Variation der Zusammensetzung der Suspension. Titandioxid C und Titandioxid G wurden dabei in einem Gewichtsverhältnis von 85:15 eingesetzt. Nach Kalzination des Katalysators für eine Stunde bei 450 °C betrug die auf die Steatitringe aufgebrachte Aktivmasse 8,5 %. Die analysierte Zusammensetzung der Aktivmasse bestand aus 7,95 % V₂O₅, 2,7 % Sb₂O₃, 0,31 % Cs, Rest TiO₂.

Katalysator KL3CG (BET-Oberfläche der Titandioxidmischung = 16,75 m²/g):
Die Herstellung erfolgte analog zu KL1 unter Variation der Zusammensetzung der Suspension. Titandioxid C und Titandioxid G wurden dabei in einem Gewichtsverhältnis von 95:5 eingesetzt. Nach Kalzination des Katalysators für eine Stunde bei 450 °C betrug die auf die Steatitringe aufgebrachte Aktivmasse 8,5 %. Die analysierte Zusammensetzung der Aktivmasse bestand aus 7,1 % V₂O₅, 2,4 % Sb₂O₃, 0,10 % Cs, Rest TiO₂.

Katalysator KL4CG (BET-Oberfläche der Titandioxidmischung = 23,05 m²/g):
Die Herstellung erfolgte analog zu KL1 unter Variation der Zusammensetzung der Suspension. Titandioxid C und Titandioxid G wurden dabei in einem Gewichtsverhältnis von 53:47 eingesetzt. Nach Kalzination des Katalysators für eine Stunde bei 450 °C betrug die auf die Steatitringe aufgebrachte Aktivmasse 9,1 %. Die analysierte Zusammensetzung der Aktivmasse bestand aus 20 % V₂O₅, 0,38 % P, Rest TiO₂.

Weitere Katalysatorlagen wurden nach der oben beschriebenen Vorschrift hergestellt, wobei unterschiedliche Titandioxide zum Einsatz kamen. Die Nomenklatur der Katalysatorlagen folgt dem oben beschriebenen Schema. Bei der Katalysatorlage KL3DH handelt es sich um eine Katalysatorlage mit der Zusammensetzung von KL3, bei der jedoch die Titandioxide D und H eingesetzt wurden.

### Vergleichsbeispiel 1

Katalysatortestung im Screeningreaktor: Ein 80 cm langes Eisenrohr mit einer lichten Weite von 15 mm wurde mit 66 cm Katalysator A gefüllt. Das Rohr war zur Temperaturreglung von einer Salzschmelze umgeben. Durch das Rohr wurden stündlich von oben nach unten 360 Nl/h Luft mit Beladungen an 98,5 Gew.-%igem o-Xylol von 56 g o-Xylol/Nm³ Luft geleitet. Bei einer Reaktortemperatur von 350 °C wurde eine PSA-Ausbeute von 75,2 mol-% erzielt (Die "PSA-Aubeute" bedeutet das erhaltene Phthalsäureanhydrid in Molprozent bzw. Gewichtsprozent, bezogen auf 100%iges o-Xylol).

### Beispiel 2

Das Vergleichsbeispiel 1 wurde wiederholt, wobei jedoch 66 cm Katalysator B in das Eisenrohr gefüllt wurden. Bei einer Reaktortemperatur von 343 °C und einer Beladung von 56 g/Nm³ wurde eine PSA-Ausbeute von 80,1 mol-% erzielt.

### Vergleichsbeispiele 3 bis 5 und Beispiel 6

Katalysatortestung im Modellrohrreaktor: Die katalytische Oxidation von o-Xylol zu Phthalsäureanhydrid wurde in einem salzbadgekühlten Rohrreaktor mit einem Innendurchmesser der Rohre von 25 mm durchgeführt. Von Reaktoreingang zu Reaktorausgang wurden 130 cm KL1, 70 cm KL2, 60 cm KL3 und 60 cm KL4 in ein 3,5 m langes Eisenrohr mit einer lichten Weite von 25 mm eingefüllt. Das Eisenrohr war zur Temperaturregelung von einer Salzschmelze umgeben, eine 4 mm Außendurchmesser Thermohülse mit eingebautem Zugelement diente der Katalysatortemperaturmessung. Durch das Rohr wurden stündlich von oben nach unten 4,0 Nm³ Luft mit Beladungen an 99,2 Gew.-%igem o-Xylol von 30 bis 100 g/Nm³ geleitet.

| Beispiel | Katalysatorlagen | Salzbadtemperatur [°C] | Beladung o-Xylol [g/Nm³] | PSA-Ausbeute [Gew.-%] |
|---|---|---|---|---|
| 3 | KL1CG, KL2CG, KL3CG, KL4CG | 361 | 66 | 111,2 |
| 4 | KL1DH, KL2DH, KL3DH, KL4DH | 361 | 66 | 113,0 |
| 5 | KL1EI, KL2E1, KL3EI, KL4EI | 361 | 66 | 112,6 |
| 6 | KL1FJ, KL2FJ, KL3FJ, KL4FJ | 361 | 66 | 114,1 |

Es zeigt sich, dass neben dem Schwefelgehalt der Ca-Gehalt des eingesetzten Titandioxids die PSA-Ausbeute stark beeinflusst.

## Patentansprüche

1. Katalysator für Gasphasenoxidationen, umfassend einen inerten Träger und eine darauf aufgebrachte katalytisch aktive Masse, die Vanadiumoxid und Titandioxid enthält, **dadurch gekennzeichnet, dass** das Titandioxid einen Gehalt an Schwefelverbindungen, berechnet als S, von weniger als 1000 ppm und einen Gehalt an Calciumverbindungen, berechnet als Ca, von weniger als 100 ppm und eine BET-Oberfläche von 15 bis 60 m²/g aufweist.

2. Katalysator nach Anspruch 1, **dadurch gekennzeichnet, dass** das Titandioxid einen Gehalt an Schwefelverbindungen, berechnet als S, von weniger als 500 ppm aufweist.

3. Katalysator nach Anspruch 1, **dadurch gekennzeichnet, dass** das Titandioxid einen Gehalt an Calciumverbindungen, berechnet als Ca, von weniger als 80 ppm aufweist.

4. Katalysator nach einem der vorhergehenden Ansprüche, **dadurch gekennzeichnet, dass** das Titandioxid eine BET-Oberfläche von 15 bis 45 m²/g aufweist.

5. Katalysator nach einem der vorhergehenden Ansprüche, **dadurch gekennzeichnet, dass** zumindest ein Teil des Titandioxids mit einem wässrigen Medium unter hydrothermalen Bedingungen behandelt wurde.

6. Katalysator nach einem der vorhergehenden Ansprüche, **dadurch gekennzeichnet, dass** die katalytisch aktive Masse 1 bis 40 Gew.-% Vanadiumoxid, berechnet als V₂O₅, und 60 bis 99 Gew.-% Titandioxid, berechnet als TiO₂, enthält.

7. Katalysator nach einem Anspruch 6, **dadurch gekennzeichnet, dass** die katalytisch aktive Masse bis zu 1 Gew.-% einer Cäsiumverbindung, berechnet als Cs, bis zu 1 Gew.-% einer Phosphorverbindung, berechnet als P und bis zu 10 Gew.-% Antimonoxid, berechnet als Sb₂O₃ enthält.

8. Verfahren zur Herstellung eines Katalysators für Gasphasenoxidationen, bei dem man auf einen inerten Träger eine Suspension von Titandioxid- und Vanadiumoxid-Teilchen aufbringt, **dadurch gekennzeichnet, dass** das Titandioxid einen Gehalt an Schwefelverbindungen, berechnet als S, von weniger als 1000 ppm und einen Gehalt an Calciumverbindungen, berechnet als Ca, von weniger als 100 ppm und eine BET-Oberfläche von 15 bis 60 m²/g aufweist.

9. Verfahren nach Anspruch 8, **dadurch gekennzeichnet, dass** die Suspension außerdem wenigstens eine Cäsium-, Phosphor- und/oder Antimonquelle enthält.

10. Verfahren nach Anspruch 8 oder 9, **dadurch gekennzeichnet, dass** man vor dem Aufbringen zumindest einen Teil des Titandioxids mit einem wässrigen Medium unter hydrothermalen Bedingungen behandelt.

11. Verfahren zur Herstellung von Phthalsäureanhydrid, **dadurch gekennzeichnet, dass** man einen Gasstrom, der molekularen Sauerstoff sowie o-Xylol, Naphthalin oder Gemische davon enthält, in Kontakt mit einem Katalysator nach einem der Ansprüche 1 bis 7 bringt.

## Claims

1. A catalyst for gas phase oxidations, comprising an inert support and a catalytically active material which comprises vanadium oxide and titanium dioxide and has been applied thereto, wherein the titanium dioxide has a content of sulfur compounds, calculated as S, of less than 1000 ppm and a content of calcium compounds, calculated as Ca, of less than 100 ppm and a BET surface area of 15 to 60 m²/g.

2. The catalyst according to claim 1, wherein the titanium dioxide has a content of sulfur compounds, calculated as S, of less than 500 ppm.

3. The catalyst according to claim 1, wherein the titanium dioxide has a content of calcium compounds, calculated as Ca, of less than 80 ppm.

4. The catalyst according to any of the preceding claims, wherein the titanium dioxide has a BET surface area of 15 to 45 m²/g.

5. The catalyst according to any of the preceding claims, wherein at least a portion of the titanium dioxide has been treated with an aqueous medium under hydrothermal conditions.

6. The catalyst according to any of the preceding claims, wherein the catalytically active material comprises 1 to 40% by weight of vanadium oxide, calculated as V₂O₅, and 60 to 99% by weight of titanium dioxide, calculated as TiO₂.

7. The catalyst according to claim 6, wherein the catalytically active material comprises up to 1% by weight of a cesium compound, calculated as Cs, up to 1% by weight of a phosphorus compound, calculated as P and up to 10% by weight of antimony oxide, calculated as Sb₂O₃.

8. A process for preparing a catalyst for gas phase oxidations, in which a suspension of titanium dioxide and vanadium oxide particles is applied to an inert support, wherein the titanium dioxide has a content of sulfur compounds, calculated as S, of less than 1000 ppm and a content of calcium compounds, calculated as Ca, of less than 100 ppm and a BET surface area of 15 to 60 m²/g.

9. The process according to claim 8, wherein the suspension also comprises at least one cesium, phosphorus and/or antimony source.

10. The process according to claim 8 or 9, wherein the application of at least a portion of the titanium dioxide is preceded by treatment with an aqueous medium under hydrothermal conditions.

11. A process for preparing phthalic anhydride, which comprises contacting a gas stream which comprises molecular oxygen and o-xylene, naphthalene or mixtures thereof with a catalyst according to any of claims 1 to 7.

## Revendications

1. Catalyseur pour oxydations en phase gazeuse, comprenant un support inerte et une masse catalytiquement active appliquée sur celui-ci, qui contient de l'oxyde de vanadium et du dioxyde de titane, **caractérisé en ce que** le dioxyde de titane présente une teneur en composés de soufre, calculée en tant que S, inférieure à 1 000 ppm et une teneur en composés de calcium, calculée en tant que Ca, inférieure à 100 ppm et une surface BET de 15 à 60 m²/g.

2. Catalyseur selon la revendication 1, **caractérisé en ce que** le dioxyde de titane présente une teneur en composés de soufre, calculée en tant que S, inférieure à 500 ppm.

3. Catalyseur selon la revendication 1, **caractérisé en ce que** le dioxyde de titane présente une teneur en composés de calcium, calculée en tant que Ca, inférieure à 80 ppm.

4. Catalyseur selon l'une quelconque des revendications précédentes, **caractérisé en ce que** le dioxyde de titane présente une surface BET de 15 à 45 m²/g.

5. Catalyseur selon l'une quelconque des revendications précédentes, **caractérisé en ce qu'**au moins une partie du dioxyde de titane a été traitée avec un milieu aqueux dans des conditions hydrothermales.

6. Catalyseur selon l'une quelconque des revendications précédentes, **caractérisé en ce que** la masse catalytiquement active contient 1 à 40 % en poids d'oxyde de vanadium, calculé en tant que V₂O₅, et 60 à 99 % en poids de dioxyde de titane, calculé en tant que TiO₂.

7. Catalyseur selon la revendication 6, **caractérisé en ce que** la masse catalytiquement active contient jusqu'à 1 % en poids d'un composé de césium, calculé en tant que Cs, jusqu'à 1 % en poids d'un composé de phosphore, calculé en tant que P, et jusqu'à 10 % en poids d'oxyde d'antimoine, calculé en tant que Sb₂O₃.

8. Procédé de fabrication d'un catalyseur pour oxydations en phase gazeuse, selon lequel une suspension de particules de dioxyde de titane et d'oxyde de vanadium est appliquée sur un support inerte, **caractérisé en ce que** le dioxyde de titane présente une teneur en composés de soufre, calculée en tant que S, inférieure à 1 000 ppm et une teneur en composés de calcium, calculée en tant que Ca, inférieure à 100 ppm et une surface BET de 15 à 60 m²/g.

9. Procédé selon la revendication 8, **caractérisé en ce que** la suspension contient également au moins une source de césium, de phosphore et/ou d'antimoine.

10. Procédé selon la revendication 8 ou 9, **caractérisé en ce qu'**au moins une partie du dioxyde de titane est traitée avec un milieu aqueux dans des conditions hydrothermales avant l'application.

11. Procédé de fabrication d'anhydride de l'acide phtalique, **caractérisé en ce qu'**un courant gazeux qui contient de l'oxygène moléculaire, ainsi que de l'oxylène, de la naphtaline ou leurs mélanges, est mis en contact avec un catalyseur selon l'une quelconque des revendications 1 à 7.
